(19) Europäisches Patentamt
European Patent Office
Office européen des brevets

(11) **EP 3 340 178 B1**

(12) **EUROPÄISCHE PATENTSCHRIFT**

(45) Veröffentlichungstag und Bekanntmachung des Hinweises auf die Patenterteilung:
**25.12.2019  Patentblatt 2019/52**

(51) Int Cl.:
***G06T 11/00*** *(2006.01)*

(21) Anmeldenummer: **17200756.9**

(22) Anmeldetag: **09.11.2017**

(54) **BERECHNEN EINES VIERDIMENSIONALEN DSA-DATENSATZES MIT VARIABLER RÄUMLICHER AUFLÖSUNG**

CALCULATION OF A FOUR-DIMENSIONAL DSA DATA SET WITH VARIABLE SPATIAL RESOLUTION

CALCUL D'UN ENSEMBLE DE DONNÉES DSA À QUATRE DIMENSIONS AU MOYEN DE PROCÉDÉ DE RÉSOLUTION SPATIALE VARIABLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.12.2016   DE 102016226195**

(43) Veröffentlichungstag der Anmeldung:
**27.06.2018   Patentblatt 2018/26**

(73) Patentinhaber: **Siemens Healthcare GmbH**
**91052 Erlangen (DE)**

(72) Erfinder:
• **Gehrisch, Sonja**
**90443 Nürnberg (DE)**
• **Kowarschik, Markus**
**90408 Nürnberg (DE)**
• **Rohkohl, Christopher**
**6364 Brixen im Thale (AT)**
• **Royalty, Kevin**
**Fitchburg, 53711 (US)**
• **Schafer, Sebastian**
**Madison, 53711 (US)**

(56) Entgegenhaltungen:
**DE-A1-102014 201 134     US-A1- 2014 376 791**

• **Kevin Royalty: "4D DSA: New Methods and Applications for 3D Time-Resolved Angiography for C-arm CT Interventional Imaging", , 31. Dezember 2014 (2014-12-31), XP055369893, Ann Arbor, United States ISBN: 978-1-321-16653-8 Gefunden im Internet: URL:http://search.proquest.com/docview/1615085787/abstract/A462590FFB214500PQ/1 [gefunden am 2017-05-05]**

**Beschreibung**

In der digitalen Subtraktionsangiographie (kurz DSA) werden ein oder mehrere Gefäße durch Röntgenaufnahmen dargestellt, wobei zur Unterdrückung von weiteren Strukturen im Untersuchungsvolumen Aufnahmen des Gefäßes alleine mit Aufnahmen des Gefäßes einschließlich eines Kontrastmittels, welches sich im Gefäß befindet, kombiniert werden. Das Kontrastmittel wird hierbei während der Untersuchung in das Gefäß eingebracht, um Parameter, insbesondere hydrodynamische Parameter eines Fluids zu bestimmen, wobei das Fluid im Gefäß fließt.

In der vierdimensionalen DSA wird mittels eines Bildrekonstruktionsverfahrens eine zeitaufgelöste Serie von dreidimensionalen DSA-Bilddaten bereitgestellt. Hierbei werden normierte zweidimensionale Röntgenprojektionen eines Untersuchungsvolumens zusammen mit einer Zeitinformation in ein Volumenelement rückprojiziert. Die zweidimensionalen Röntgenprojektionen entstammen hierbei üblicherweise einem rotierenden Aufnahmeprotokoll eines C-Arm-Röntgenbogens. Grundlagen der vierdimensionalen DSA sind beispielsweise in der Dissertationsschrift K. Royalty: "4D DSA: New Methods and Applications for 3D Time-Resolved Angiography for C-arm CT Interventional Imaging" University of Wisconsin-Madison (2014) beschrieben, weiterhin beschreibt diese Druckschrift Methoden zur Validierung der vierdimensionalen DSA.

Die multiplikative Rückprojektion unterliegt Einschränkungen, falls mehrere Gefäße oder mehrere Gefäßabschnitte in den zweidimensionalen Röntgenprojektionen überlappen. In diesem Fall ist aus einer einzelnen Röntgenprojektion nicht ersichtlich, welchem der überlappenden Gefäße ein Röntgensignal, insbesondere ein Intensitätswert oder ein Röntgenabsorptionskoeffizient zugeordnet werden muss. Dies ist insbesondere der Fall, wenn Überlappungen mit Gefäßen außerhalb des Rekonstruktionsvolumens auftreten.
Aufgrund von Standards in der medizinischen Bildgebung wie beispielsweise DICOM (englisches Akronym für "Digital Imaging and Communications in Medicine", eine deutsche Übersetzung ist "Digitale Bildverarbeitung und -kommunikation in der Medizin") ist die Anzahl von Voxeln in einem dreidimensionalen Bilddatensatz festgelegt, z.B. als 256 x 256 x 256 oder als 512 x 512 x 512.

Es ist bekannt, die multiplikative Rückprojektion für ein maximal großes Rekonstruktionsvolumen durchzuführen, um einen möglichen Überlapp von Gefäßen bestmöglich aufzulösen. Aufgrund der durch Standards festgelegten Voxelzahl ist damit aber durch die Größe des Rekonstruktionsvolumens die räumliche Auflösung begrenzt.

Weiterhin ist bekannt, die multiplikative Rückprojektion für ein kleineres Rekonstruktionsvolumen durchzuführen. Hierbei verbessert sich zwar die Auflösung, es können aber Gefäße oder Gefäßabschnitte außerhalb des Rekonstruktionsvolumens die Ergebnisse verfälschen.

Es ist daher die Aufgabe der vorliegenden Erfindung, unter Berücksichtigung von Gefäßen außerhalb des Rekonstruktionsvolumens eine variable, insbesondere eine feinere räumliche Auflösung des Rekonstruktionsvolumens zu erreichen.

Die Aufgabe wird gelöst durch ein Verfahren nach Anspruch 1, durch eine DSA-Berechnungseinheit nach Anspruch 11, durch eine Röntgeneinheit nach Anspruch 13, durch ein Computerprogrammprodukt nach Anspruch 14 sowie durch ein computerlesbares Speichermedium nach Anspruch 15.

Nachstehend wird die erfindungsgemäße Lösung der Aufgabe in Bezug auf die beanspruchten Vorrichtungen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Die Erfindung basiert darauf, dass ein Untersuchungsvolumen betreffende Röntgendatensätze mittels einer Schnittstelle empfangen werden, wobei jeder der Röntgendatensätze eine zweidimensionale Röntgenprojektion des Untersuchungsvolumens bezüglich einer Projektionsrichtung und einen Aufnahmezeitpunkt der Röntgenprojektion umfasst. Hierbei umfasst das Untersuchungsvolumen wenigstens ein Gefäß, wobei das Gefäß ein Kontrastmittel enthalten kann, und wobei sich die räumliche Dichte des Kontrastmittels mit der Zeit ändern kann. Der Aufnahmezeitpunkt entspricht dem Zeitpunkt der Aufnahme der zweidimensionalen Röntgenprojektion. Eine zweidimensionale Röntgenprojektion ist insbesondere räumlich zweidimensional. Bei den Röntgenprojektionen kann es sich insbesondere auch um DSA-Röntgenprojektionen handeln.

Die Erfindung basiert weiterhin darauf, dass ein erster dreidimensionaler DSA-Datensatz eines ersten Rekonstruktionsvolumens basierend auf den Röntgendatensätzen mittels einer Recheneinheit bestimmt wird, wobei das erste Rekonstruktionsvolumen ein Teil des Untersuchungsvolumens oder mit diesem identisch ist. Ein dreidimensionaler Datensatz ist insbesondere räumlich dreidimensional.

Die Erfindung basiert weiterhin darauf, dass ein zweiter dreidimensionaler DSA-Datensatz eines zweiten Rekonstruktionsvolumens basierend auf den Röntgendatensätzen mittels der Recheneinheit bestimmt wird, wobei das zweite Rekonstruktionsvolumen ein Teil des ersten Rekonstruktionsvolumens ist. Hierbei basiert das Bestimmen des zweiten dreidimensionalen DSA-Datensatzes insbesondere nur auf den Röntgendatensätzen, also insbesondere nicht auf dem ersten dreidimensionalen DSA-Datensatz.

Die Erfindung basiert weiterhin darauf, dass der zweite dreidimensionale DSA-Datensatz mittels der Recheneinheit segmentiert wird. Hierbei wird der DSA-Datensatz in wenigstens zwei Teile segmentiert, wobei ein erster Teil wenigstens ein im zweiten Rekonstruktionsvolumen enthaltenes Gefäß und das Innere des Gefäßes umfasst, und ein zweiter Teil die anderen Bestandteile des zweiten Rekonstruktionsvolumens umfasst. Der erste Teil kann auch mehrere im zweiten Rekonstruktionsvolumen enthaltene Gefäße und das Innere der Gefäße umfassen.

Die Erfindung basiert weiterhin darauf, dass die Röntgendatensätze basierend auf dem ersten dreidimensionalen DSA-Datensatz mittels der Recheneinheit normiert werden.

Die Erfindung basiert weiterhin darauf, dass ein vierdimensionaler DSA-Datensatz durch Rückprojektion der normierten Röntgendatensätze auf den segmentierten zweiten dreidimensionalen DSA-Datensatz mittels der Recheneinheit berechnet wird, wobei der vierdimensionale DSA-Datensatz mehrere dritte dreidimensionale DSA-Datensätze sowie eine zugehörige Zeitinformation umfasst. Hierbei ist jedem der dritten dreidimensionalen DSA-Datensätze eine Zeitinformation zugeordnet. Hierbei entspricht die zu einem dritten dreidimensionalen DSA-Datensatz zugehörige Zeitinformation insbesondere der Zeit, zu der der Zustand des dargestellten Gefäßes der Abbildung im dreidimensionalen DSA-Datensatz entspricht. Insbesondere kann auch jeder der Voxel jedes der dritten dreidimensionalen DSA-Datensätze eine Zeitinformation umfassen. Eine Zeitinformation kann insbesondere auch eine Zeitkoordinate sein. Eine Rückprojektion kann insbesondere eine multiplikative Rückprojektion sein.

Die Erfinder haben erkannt, dass durch das erste Bestimmen eines ersten dreidimensionalen DSA-Datensatzes und durch das zweite Bestimmen eines zweiten dreidimensionalen DSA-Datensatzes das Normieren auf dem ersten dreidimensionalen DSA-Datensatz basieren kann und das Segmentieren sowie das Berechnen des vierdimensionalen DSA-Datensatzes auf dem zweiten dreidimensionalen DSA-Datensatz basieren können. Da das erste Rekonstruktionsvolumen größer ist als das zweite Rekonstruktionsvolumen, kann eine variable, insbesondere eine bessere räumliche Auflösung des zweiten dreidimensionalen DSA-Datensatzes und gleichzeitig Informationen über weitere in den Röntgenprojektionen abgebildete Gefäße aus dem größeren ersten Rekonstruktionsvolumen verwendet werden.

Nach einem weiteren Aspekt der Erfindung wird jeder dritte dreidimensionale DSA-Datensatz des vierdimensionalen DSA-Datensatzes durch Rückprojektion von genau einem der zweidimensionalen Röntgendatensätze berechnet, wobei die zugehörige Zeitinformation dem Aufnahmezeitpunkt des zweidimensionalen Röntgendatensatzes entspricht. Die Erfinder haben erkannt, dass durch die eindeutige Zuordnung einer Röntgenprojektion zu einem dreidimensionalen DSA-Datensatz die zum dreidimensionalen DSA-Datensatz zugehörige Zeitinformation eindeutig und damit fehlerfrei bestimmt werden kann.

Nach einem weiteren Aspekt der Erfindung umfasst das Verfahren weiterhin ein drittes Bestimmen eines Konfidenzwertes für wenigstens ein erstes Pixel wenigstens einer der Röntgenprojektionen basierend auf dem ersten dreidimensionalen DSA-Datensatz, ein Zuordnen des Konfidenzwertes zu einem ersten Voxel wenigstens eines der dritten dreidimensionalen DSA-Datensätze, wobei der Wert des ersten Voxels auf dem Wert des ersten Pixels basiert, sowie ein Interpolieren des vierdimensionalen DSA-Datensatzes basierend auf dem Konfidenzwert. Der Wert des ersten Voxels basiert insbesondere auf dem Wert des ersten Pixels, wenn der Wert des ersten Voxel durch Rückprojektion aus dem Wert des ersten Pixels berechnet wird. Hierbei werden das dritte Bestimmen, das Zuordnen und das Interpolieren jeweils mittels der Recheneinheit durchgeführt. Die Erfinder haben erkannt, dass es durch eine Interpolation möglich ist, valide und weniger fehleranfällige vierdimensionale DSA-Datensätze auch bei Gefäßen oder Gefäßteilen zu ermitteln, deren Röntgenprojektionen bezüglich einer oder mehrerer Projektionsrichtungen an einem oder mehreren Zeitpunkten überlappen.

Nach einem weiteren möglichen Aspekt der Erfindung basiert ein interpolierter Intensitätswert eines Voxels eines ersten der dritten dreidimensionalen DSA-Datensätze nur auf den Intensitätswerten des entsprechenden Voxels in den dritten dreidimensionalen DSA-Datensätzen. Dabei ist ein entsprechendes Voxel insbesondere ein räumlich entsprechendes Voxel. Die Erfinder haben erkannt, dass der Gradient der Konzentration eines Kontrastmittels bezüglich der Zeit kleiner und gleichmäßiger ist als bezüglich einer der drei Raumrichtungen, und dass daher eine zeitliche Interpolation besonders gute Ergebnisse erzeugt. Insbesondere basiert der interpolierte Intensitätswert des ersten Voxels nur auf den Intensitätswerten eines dem ersten Voxel entsprechenden zweiten Voxels in einem zweiten der dritten dreidimensionalen DSA-Datensätze und eines dem ersten Voxel entsprechenden dritten Voxels in einem dritten der dritten dreidimensionalen DSA-Datensätze. Insbesondere wird der interpolierte Intensitätswert durch eine lineare Interpolation bestimmt. Die Erfinder haben erkannt, dass eine lineare Interpolation besonders einfach durchführbar und robust bezüglich einer Überanpassung ist.

Nach einem weiteren Aspekt der Erfindung sinkt der Konfidenzwert des ersten Pixels einer Röntgenprojektion monoton mit einer Anzahl der auf den ersten Pixel projizierten Gefäßabschnitte in dem ersten dreidimensionalen DSA-Datensatz, wobei die Gefäßabschnitte in die Projektionsrichtung der Röntgenprojektion projiziert werden. Die Erfinder haben erkannt, dass die Verlässlichkeit von rückprojizierten dreidimensionalen Daten mit der Anzahl der in einer Röntgenprojektion überlappenden Gefäßabschnitte abnimmt, und daher die Anzahl der überlappenden Gefäßabschnitte ein geeignetes Kriterium für die Verlässlichkeit oder Exaktheit eines Pixelwertes ist.

Nach einem weiteren Aspekt der Erfindung betrifft das Interpolieren Voxel, denen ein Konfidenzwert kleiner als ein Schwellenwert zugeordnet ist. Die Erfinder haben erkannt, dass anhand des Schwellenwerts besonders schnell und einfach Voxel ausgewählt werden können, bei denen aufgrund von Überlappung keine gesicherte Information über den Intensitätswert möglich ist. Der Schwellenwert kann insbesondere so gewählt sein, dass alle Voxel mit einem Konfidenzwert kleiner als der maximal zugewiesene Konfidenzwert interpoliert werden. Hierdurch werden die Intensitätswerte aller Voxel, die nicht alleine durch die zugehörige Röntgenprojektion bestimmt werden können und daher eine Unsicherheit oder falsche Daten aufweisen, durch Interpolation verbessert oder berichtigt.

Nach einem weiteren Aspekt der Erfindung umfassen der erste und der zweite dreidimensionale DSA-Datensatz jeweils homogene Voxel. Die Voxel eines DSA-Datensatzes sind insbesondere homogen, wenn alle Paare von zwei Voxeln aus dem DSA-Datensatz bezüglich einer zu einer ersten Voxelkante parallelen ersten Achse, bezüglich einer zur einer zweiten Voxelkante parallelen und zur ersten Achse orthogonalen zweiten Achse und bezüglich einer zu einer dritten Voxelkante parallelen und zur ersten Achse und zur zweiten Achse orthogonalen dritten Achse jeweils die gleiche räumliche Ausdehnung aufweisen. Die Voxel eines DSA-Datensatzes können insbesondere auch isotrop sein, also jedes Voxel eines DSA-Datensatzes bezüglich der ersten Achse, der zweiten Achse und der dritten Achse die gleiche Ausdehnung aufweisen. Der Wert eines Voxels kann insbesondere ein Röntgenabsorptionskoeffizient oder ein binärer Wert sein, wobei der binäre Wert die Zugehörigkeit eines Voxels zu einer bestimmten Struktur bezeichnet. Die Erfinder haben erkannt, dass bei homogenen Voxeln das Verfahren besonders schnell und effizient durchgeführt werden kann, da die Rückprojektion nicht an die Geometrie einzelner Voxel angepasst werden muss und daher vektorisiert und parallel berechnet werden kann.

Nach einem weiteren Aspekt der Erfindung entspricht die Orientierung der Voxel im ersten dreidimensionalen DSA-Datensatz der Orientierung der Voxel im zweiten dreidimensionalen DSA-Datensatz. Die Orientierung eines ersten Voxels im ersten dreidimensionalen DSA-Datensatz entspricht insbesondere der Orientierung eines zweiten Voxels im zweiten dreidimensionalen DSA-Datensatz, wenn jede Kante des ersten Voxels parallel zu einer Kante des zweiten Voxels ist. Die Erfinder haben erkannt, dass sich durch die gleiche Orientierung der Voxel rechnerische Vereinfachungen im Verfahren ergeben, die eine besonders schnelle und effiziente Ausführung des Verfahrens erlauben.

Nach einem weiteren Aspekt der Erfindung ist die Länge der bezüglich einer ersten Koordinatenachse parallelen Kanten der Voxel des zweiten dreidimensionalen DSA-Datensatzes kleiner als die Länge der bezüglich der ersten Koordinatenachse parallelen Kanten der Voxel des ersten dreidimensionalen DSA-Datensatzes. Insbesondere kann auch die Länge der zu einer zweiten oder dritten Koordinatenachse parallelen Kanten der Voxel des zweiten dreidimensionalen DSA-Datensatzes kleiner sein als die Länge der zu einer zweiten oder dritten Koordinatenachse parallelen Kanten der Voxel des ersten dreidimensionalen DSA-Datensatzes. Die Erfinder haben erkannt, dass durch ein solches Verhältnis der Kantenlängen die räumliche Auflösung des resultierenden vierdimensionalen DSA-Datensatzes verbessert werden kann.

Nach einem weiteren Aspekt der Erfindung ist die Zahl der Voxel im ersten dreidimensionalen DSA-Datensatz gleich der Zahl der Voxel im zweiten dreidimensionalen DSA-Datensatz. Die Erfinder haben erkannt, dass durch die gleiche Zahl der Voxel im ersten und im zweiten dreidimensionalen DSA-Datensatz beide DSA-Datensätze in einer gleichartigen Datenstruktur, insbesondere in einem DICOM-Datensatz gespeichert werden können. Dies vereinfacht die Speicher- und Datenverwaltung und ermöglicht einen standardisierten Datenaustausch.

Nach einem weiteren Aspekt der Erfindung sind die Kantenlängen der Voxel des zweiten dreidimensionalen DSA-Datensatzes größer als die Kantenlänge der Pixel der Röntgendatensätze. Die Erfinder haben erkannt, dass durch ein derartiges Verhältnis der Kantenlängen ein Pixel eines der Röntgenprojektionen der Röntgendatensätze höchstens einem Voxel des zweiten dreidimensionalen DSA-Datensatzes zugeordnet ist. Dadurch ist der Wert eines Voxels immer genau durch den Wert wenigstens eines Pixels bestimmt und muss nicht durch eine Interpolation bestimmt werden.

Die Erfindung betrifft weiterhin eine DSA-Berechnungseinheit zum Berechnen eines vierdimensionalen DSA-Datensatzes, umfassend folgende Einheiten:

- Schnittstelle, ausgebildet zum Empfangen von Röntgendatensätzen betreffend ein Untersuchungsvolumen, wobei jeder der Röntgendatensätze eine zweidimensionale Röntgenprojektion des Untersuchungsvolumens bezüglich einer Projektionsrichtung und einen Aufnahmezeitpunkt der Röntgenprojektion umfasst,
- Recheneinheit, ausgebildet zum ersten Bestimmen eines ersten dreidimensionalen DSA-Datensatzes eines ersten Rekonstruktionsvolumens basierend auf den Röntgendatensätzen, wobei das erste Rekonstruktionsvolumen ein Teil des Untersuchungsvolumens oder mit diesem identisch ist,

weiterhin ausgebildet zum zweiten Bestimmen eines zweiten dreidimensionalen DSA-Datensatzes eines zweiten Rekonstruktionsvolumens basierend auf den Röntgendatensätzen, wobei das zweite Rekonstruktionsvolumen ein Teil des ersten Rekonstruktionsvolumens ist,
weiterhin ausgebildet zum Segmentieren des zweiten dreidimensionalen DSA-Datensatzes,

weiterhin ausgebildet zum Normieren der Röntgendatensätze basierend auf dem ersten dreidimensionalen DSA-Datensatz weiterhin ausgebildet zum Berechnen eines vierdimensionalen DSA-Datensatzes durch Rückprojektion der normierten Röntgendatensätze auf den segmentierten zweiten dreidimensionalen DSA-Datensatz, wobei der vierdimensionale DSA-Datensatz mehrere dritte dreidimensionale DSA-Datensätze sowie eine zugehörige Zeitinformation umfasst.

Eine solche DSA-Berechnungseinheit kann insbesondere dazu ausgebildet sein die zuvor beschriebenen erfindungsgemäßen Verfahren und ihre Aspekte auszuführen. Die DSA-Berechnungseinheit ist dazu ausgebildet diese Verfahren und ihre Aspekte auszuführen, indem die Schnittstelle und die Recheneinheit dazu ausgebildet sind, die entsprechenden Verfahrensschritte auszuführen. Die Erfindung betrifft weiterhin eine Röntgeneinheit, ausgebildet zum Aufnehmen von Röntgendatensätzen sowie umfassend eine erfindungsgemäße DSA-Berechnungseinheit.

Die Erfindung betrifft auch ein Computerprogrammprodukt mit einem Computerprogramm sowie ein computerlesbares Medium. Eine weitgehend softwaremäßige Realisierung hat den Vorteil, dass auch schon bisher verwendete DSA-Berechnungseinheiten auf einfache Weise durch ein Software-Update nachgerüstet werden können, um auf die erfindungsgemäße Weise zu arbeiten. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen.

Eine Röntgenprojektion ist eine zweidimensionale Projektion eines Untersuchungsvolumens mittels Röntgenstrahlen entlang einer Projektionsrichtung, die insbesondere mehrere Pixel umfassen kann. Dabei wird jedem Pixel ein Röntgenintensitätswert zugewiesen, der ein Maß für die in diesem Pixel aufgetroffene Röntgenintensität ist. Die auftreffende Röntgenintensität hängt von der Zahl, der Größe, der Form und dem Material der sich im Untersuchungsvolumen befindlichen Objekte ab. Eine Kantenlänge einer Kante eines Pixels ist die Länge im Untersuchungsvolumen, die der Kante des Pixels entspricht.

Eine DSA-Röntgenprojektion eines Untersuchungsvolumens kann aus einer ersten Röntgenprojektion und einer zweiten Röntgenprojektion des Untersuchungsvolumens bestimmt werden, wobei die erste Röntgenprojektion und die zweite Röntgenprojektion bezüglich der gleichen Projektionsrichtung aufgenommen wurden, und wobei zum Zeitpunkt der Aufnahme der ersten Röntgenprojektion eine andere Kontrastmittelverteilung im Untersuchungsvolumen als zum Zeitpunkt der Aufnahme der zweiten Röntgenprojektion vorgelegen hat. Die DSA-Röntgenprojektion kann dann aus der Differenz der Röntgenintensitäten der ersten Röntgenprojektion und der zweiten Röntgenprojektion berechnet werden.

Aus mehreren Röntgenprojektionen aus unterschiedlichen Projektionsrichtungen kann ein dreidimensionaler Datensatz des Untersuchungsvolumens rekonstruiert werden. Handelt es sich bei den mehreren Röntgenprojektionen um DSA-Röntgenprojektionen, kann ein dreidimensionaler DSA-Datensatz des Untersuchungsvolumens rekonstruiert werden. Ein dreidimensionaler Datensatz oder ein dreidimensionaler DSA-Datensatz kann insbesondere mehrere Voxel umfassen, denen eine Röntgenabsorption oder eine Röntgenintensität zugeordnet ist. Die Röntgenabsorption kann in Hounsfield-Einheiten (ein englischer Fachbegriff ist "Hounsfield-Units", kurz "HU") gemessen werden.

Ein vierdimensionaler DSA-Datensatz umfasst mehrere dreidimensionale Voxel, denen eine Zeitinformation zugeordnet ist. Äquivalent kann ein vierdimensionaler DSA-Datensatz auch dadurch beschrieben werden, dass er mehrere dreidimensionale DSA-Datensätze umfasst, wobei einem dreidimensionalen DSA-Datensatz eine Zeitinformation zugeordnet ist. Eine Zeitinformation kann als Zeitkoordinate aufgefasst werden, und der vierdimensionale DSA-Datensatz kann als zeitliche Abfolge oder Film von dreidimensionalen DSA-Datensätzen aufgefasst werden.

Eine Rückprojektion ist ein Verfahren, das aus einem oder mehreren zweidimensionalen Projektionen eines dreidimensionalen Untersuchungsvolumens Daten betreffend das dreidimensionale Untersuchungsvolumen ermittelt. Bei den Daten betreffend das dreidimensionale Untersuchungsvolumen kann es sich insbesondere um Absorptionskoeffizienten oder Hounsfield-Einheiten handeln. Da eine zweidimensionale Projektion weniger Informationen enthält als das dreidimensionale Untersuchungsvolumen, können für eine Rückprojektion weitere Informationen verwendet werden, beispielsweise eine Segmentierung des Untersuchungsvolumens oder eines Rekonstruktionsvolumens.

Im Folgenden wird die Erfindung anhand der in den Figuren dargestellten Ausführungsbeispiele näher beschrieben und erläutert.

Es zeigen

Fig. 1    ein Flussdiagramm eines Verfahrens zum Berechnen eines vierdimensionalen DSA-Datensatzes aus Röntgendatensätzen,

Fig. 2    eine DSA-Berechnungseinheit,

Fig. 3    eine Röntgeneinheit mit DSA-Berechnungseinheit,

Fig. 4    ein erstes und ein zweites Gefäß in einem ersten Untersuchungsvolumen,

Fig. 5    Röntgendatensätze umfassend jeweils eine Röntgenprojektion des ersten und des zweiten Gefäßes.

Der erste Schritt des dargestellten Ausführungsbeispiels des Verfahrens ist das Empfangen REC von Röntgendatensätzen 500 betreffend ein Untersuchungsvolumen 400 mittels einer Schnittstelle 201, wobei jeder der Röntgendatensätze 500 eine zweidimensionale Röntgenprojektion 501.1, ..., 501.4 des Untersuchungsvolumens 400 bezüglich einer Projektionsrichtung und einen Aufnahmezeitpunkt der Röntgenprojektion 501.1, ..., 501.4 umfasst.

Im dargestellten Ausführungsbeispiel wurden die Röntgenprojektionen 501.1, ..., 501.4 mit einem C-Bogen-Röntgengerät 300 aufgenommen. Hierbei werden für jede Projektionsrichtung genau zwei Röntgendatensätze 500 aufgenommen, wobei bei dem jeweils ersten Röntgendatensatz aus jeder Projektionsrichtung kein Röntgenkontrastmittel im ersten Gefäß 403 vorhanden ist, und wobei bei dem jeweils zweiten Röntgendatensatz Röntgenkontrastmittel im ersten Gefäß 403 vorhanden ist. Dabei werden die Röntgendatensätze ohne Röntgenkontrastmittel derart aufgenommen, dass der C-Bogen 303 einen vorgegebenen Winkel um das Untersuchungsvolumen 400 rotiert und im konstanten zeitlichen Abstand Röntgenprojektionen aufgenommen werden. Weiterhin werden die Röntgendatensätze 500 mit Röntgenkotrastmittel derart aufgenommen, dass der C-Bogen 303 den vorgegebenen Winkel um das Untersuchungsvolumen 400 rotiert und dabei im gleichen konstanten zeitlichen Abstand Röntgenprojektionen 501.1, ..., 501.4 aufgenommen werden.

Bei jeder Aufnahmeserie rotiert der C-Bogen 303 des C-Bogen-Röntgengeräts 300 in 12 Sekunden um 260° und nimmt dabei 304 Röntgenprojektionen aus unterschiedlichen Projektionsrichtungen auf. Es sind auch Aufnahmeparameter umfassend andere Rotationswinkel, Rotationszeiträume und Projektionszahlen möglich, insbesondere solche Aufnahmeparameter, die zu Röntgendatensätzen führen, die zu einer dreidimensionalen Rekonstruktion geeignet sind. Hierbei sind insbesondere Rotationswinkel geeignet, die größer sind als die Summe von 180° und dem Öffnungswinkel der Röntgenstrahlen der Röntgenquelle 301, insbesondere Rotationswinkel größer als 200°. Bei der Aufnahme der Röntgenprojektionen 501.1, ..., 501.4 mit Kontrastmittel kann der C-Bogen 303 im gleichen Umlaufsinn wie bei der Aufnahme der Röntgenprojektionen ohne Kontrastmittel rotieren. In diesem Fall muss der C-Bogen 303 zwischen den Aufnahmen in die Ausgangsposition zurückfahren. Der C-Bogen 303 kann aber auch im entgegengesetzten Umlaufsinn wie bei der Aufnahme der Röntgenprojektionen ohne Kontrastmittel rotieren.

Aus jeweils einem ersten Röntgendatensatz ohne Kontrastmittel und einem zweiten Röntgendatensatz mit Kontrastmittel, wobei die Röntgenprojektionen des ersten und des zweiten Röntgendatensatzes aus der gleichen Projektionsrichtung aufgenommen wurden, kann dann durch Subtraktion der Intensitätswerte der Röntgenprojektion des zweiten Röntgendatensatzes von der Röntgenprojektion des ersten Röntgendatensatzes eine zweidimensionale DSA-Röntgenprojektion ermittelt werden.

Es ist aber alternativ auch möglich, das in diesem Schritt des Verfahrens direkt Röntgendatensätze 400 umfassend DSA-Röntgenprojektionen empfangen werden.

Weitere Schritte des dargestellten Ausführungsbeispiels des Verfahrens sind das erste Bestimmen DET-1 eines ersten dreidimensionalen DSA-Datensatzes eines ersten Rekonstruktionsvolumens 401 basierend auf den Röntgendatensätzen 500 mittels einer Recheneinheit 202, wobei das erste Rekonstruktionsvolumen 401 ein Teil des Untersuchungsvolumens 400 oder mit diesem identisch ist, und das zweite Bestimmen DET-2 eines zweiten dreidimensionalen DSA-Datensatzes eines zweiten Rekonstruktionsvolumens 402 basierend auf den Röntgendatensätzen 500 mittels der Recheneinheit 202, wobei das zweite Rekonstruktionsvolumen 402 ein Teil des ersten Rekonstruktionsvolumens 401 ist. Hierbei basiert der zweite dreidimensionale DSA-Datensatz insbesondere nicht auf dem ersten dreidimensionalen DSA-Datensatz. Weiterhin ist die Reihenfolge des Ausführens des ersten Bestimmens DET-1 und des zweiten Bestimmens DET-2 nicht relevant.

Im gezeigten Ausführungsbeispiel basieren das erste Bestimmen DET-1 und das zweite Bestimmen DET-2 jeweils auf den aus einer Subtraktion ermittelten zweidimensionalen DSA-Röntgenprojektionen. Das erste Bestimmen DET-1 und das zweite Bestimmen DET-2 können insbesondere nur auf denjenigen zweidimensionalen DSA-Röntgenprojektionen basieren, bei denen das zu untersuchende erste Gefäß 403 komplett oder zu großen Teilen mit Kontrastmittel gefüllt ist.

Im dargestellten Ausführungsbeispiel wird erste dreidimensionale DSA-Datensatz und der zweite dreidimensionale DSA-Datensatz mittels einer Kegelstrahl-Rekonstruktion (ein englischer Fachbegriff ist "cone beam reconstruction") aus den zweidimensionalen Röntgenprojektionen oder den zweidimensionalen DSA-Röntgenprojektionen bestimmt. Es sind aber auch andere Rekonstruktionsverfahren möglich, beispielsweise eine Fächerstrahlrekonstruktion (ein englischer Fachbegriff ist "fan beam reconstruction"). Hierbei werden die Hounsfield-Units des jeweiligen Rekonstruktionsvolumens bestimmt.

Im dargestellten Ausführungsbeispiel ist das erste Rekonstruktionsvolumen 401 identisch mit dem Untersuchungsvolumen 400. Das erste Rekonstruktionsvolumen 401 kann aber auch kleiner sein als das Untersuchungsvolumen 400 und im Untersuchungsvolumen 400 enthalten sein. Das zweite Rekonstruktionsvolumen 402 ist kleiner als das erste Rekonstruktionsvolumen 401 und Teil des ersten Rekonstruktionsvolumens 401. Im dargestellten Ausführungsbeispiel sind das erste Rekonstruktionsvolumen 401 und das zweite Rekonstruktionsvolumen 402 würfelförmig ausgebildet. Es sind aber für die Rekonstruktionsvolumina 401, 402 auch andere Geometrien, insbesondere eine quaderförmige Geometrie, vorstellbar.

Im dargestellten Ausführungsbeispiel bestehen sowohl der erste dreidimensionale DSA-Datensatz als auch der zweite dreidimensionale DSA-Datensatz aus der gleichen Anzahl an isotropen Voxeln, und zwar aus 512 x 512 x 512 Voxeln, was dem DICOM-Standard entspricht. Es sind aber auch andere, insbesondere auch abweichende Voxelzahlen möglich, insbesondere auch 256 x 256 x 256 Voxel, was ebenfalls einem DICOM-Standard entspricht. Es sind weiterhin nicht-isotrope Voxel möglich. Durch die gleiche Voxelzahl und das unterschiedliche Rekonstruktionsvolumen 401, 402 hat der zweite dreidimensionale DSA-Datensatz eine bessere räumliche Auflösung als der erste dreidimensionale DSA-Datensatz. Weiterhin weisen die Voxel des ersten dreidimensionalen DSA-Datensatzes die gleiche Ausrichtung wie die Voxel des zweiten dreidimensionalen DSA-Datensatzes auf. Sie können aber auch andere Ausrichtungen aufweisen.

Ein weiterer Schritt des dargestellten Ausführungsbeispiels des Verfahrens ist das Segmentieren SEG des zweiten DSA-Datensatzes mittels der Recheneinheit 202. Im dargestellten Ausführungsbeispiel erfolgt das Segmentieren SEG mittels einer Schwellenwert-Segmentierung, es werden also alle Voxel des zweiten DSA-Datensatzes mit Hounsfield-Einheiten über dem Schwellenwert einer ersten Region zugeordnet, die hier einem ersten Gefäß 403 entspricht, weiterhin werden alle Voxel des zweiten DSA-Datensatzes mit Hounsfield-Einheiten unter dem Schwellenwert einer zweiten Region zugeordnet. Es sind aber auch andere Methoden zur Segmentierung möglich, beispielsweise Regionenwachstum (ein englischer Fachbegriff ist "region growing") oder aktive Formmodelle (ein englischer Fachbegriff ist "active shape models"). Das Ergebnis der Segmentierung kann als Funktion $C_2$ aufgefasst werden, wobei die Funktion $C_2$ einem Voxel mit der räumlich dreidimensionalen Koordinate x einen Wert $C_2(x)$ zuordnet, falls der Voxel in der ersten Region liegt, wobei der Wert $C_2(x)$ dem Wert des Voxels im zweiten DSA-Datensatz entspricht, und wobei die Funktion $C_2$ einem Voxel mit der räumlich dreidimensionalen Koordinate x einen Wert $C_2(x) = 0$ zuordnet, falls der Voxel in der zweiten Region liegt.

Ein weiterer Schritt des dargestellten Ausführungsbeispiels des Verfahrens ist das Normieren NORM der Röntgendatensätze 500 basierend auf dem ersten dreidimensionalen DSA-Datensatz mittels der Recheneinheit 202, wobei die Normierung in diesem Ausführungsbeispiel durch den folgenden funktionalen Zusammenhang gegeben ist:

$$p_N(t,u) = \frac{p(t,u)}{\int_{L(t,u)} I_1(l)\, dl}$$

Hierbei ist u eine zweidimensionale räumliche Koordinate im Koordinatensystem des Röntgendetektors 302, und t ist eine zeitliche Koordinate, insbesondere also eine Zeitinformation.

Weiterhin bezeichnet $I_1(l)$ den Intensitätswert des ersten dreidimensionalen DSA-Datensatzes an einer dreidimensional räumlichen Koordinate l. Der eindimensionale Pfad $L(t,u)$ entspricht der Gerade durch die punktförmige Röntgenquelle 301 und den Punkt u auf dem Röntgendetektor 302 zum Aufnahmezeitpunkt t. Der Pfad $L(t,u)$ ist weiterhin von der zeitlichen Koordinate t abhängig, weil sich die räumliche Position der Röntgenquelle 301 und des Röntgendetektors 302 mit der zeitlichen Koordinate t verändern. Die Größe $p(t,u)$ beschreibt den Intensitätswert der zum Aufnahmezeitpunkt t aufgenommenen Röntgenprojektion in der Detektorkoordinate u. Das Ergebnis $p_N(t,u)$ ist der normierte Intensitätswert der zum Zeitpunkt t aufgenommenen Röntgenprojektion in der Detektorkoordinate u.

Ein weiterer Schritt des dargestellten Ausführungsbeispiels des Verfahrens ist das Berechnen CALC eines vierdimensionalen DSA-Datensatzes durch Rückprojektion der normierten Röntgendatensätze 500 auf den segmentierten zweiten dreidimensionalen DSA-Datensatz mittels der Recheneinheit 202, wobei der vierdimensionale DSA-Datensatz mehrere dritte dreidimensionale DSA-Datensätze sowie zugehörige Zeitinformationen umfasst. In diesem Ausführungsbeispiel wird eine multiplikative Rückprojektion verwendet, die durch folgenden funktionalen Zusammenhang gegeben ist:

$$f(t,x) = C_2(x)\, \frac{p_N(t,\ A(t,x))}{K * \int_{L(t,\ A(t,x))} I_2(l)\, dl}$$

Hierbei ist x eine dreidimensionale räumliche Koordinate und t eine zeitliche Koordinate, insbesondere also eine Zeitinformation. Das Tupel (t,x) kann daher auch als vierdimensionale Koordinate aufgefasst werden. Weiterhin bezeichnet $I_2(x)$ den zweiten dreidimensionalen DSA-Datensatz. $A(t,x)$ bezeichnet die Projektion der räumlichen Koordinate x zum Aufnahmezeitpunkt t auf die räumlich zweidimensionale Detektorkoordinate u = $A(t,x)$. Weiterhin bezeichnet K einen optionalen Faltungskern, der Operator * eine Faltung und $C_2(x)$ bezeichnet die zur Segmentierung des zweiten dreidimensionalen DSA-Datensatzes gehörige Funktion. Weiterhin bezeichnet f(t,x) den vierdimensionalen DSA-Datensatz, der mehrere dritte dreidimensionale DSA-Datensätze $f(t_1, x)$, ..., $f(t_N, x)$ umfasst.

Ein weiterer optionaler Schritt des dargestellten Ausführungsbeispiels des Verfahrens ist ein drittes Bestimmen DET-3 eines Konfidenzwertes für wenigstens ein erstes Pixel wenigstens einer der Röntgenprojektionen 501.1, 501.2, 501.3, 501.4 basierend auf dem ersten dreidimensionalen DSA-Datensatz. Im dargestellten Ausführungsbeispiel ist der Konfidenzwert eines Pixels einer der Röntgenprojektionen 501.1, 501.2, 501.3, 501.4 ein Maß für die Zahl der Gefäße 403, 404 und/oder Gefäßabschnitte 403.1, 403.2, die auf das Pixel abgebildet werden. Der Konfidenzwert ist hier 1, wenn nur genau ein Gefäß 403, 404 und/oder Gefäßabschnitt 403.1, 403.2 auf das Pixel abgebildet wird, ansonsten sinkt der Konfidenzwert mit der Zahl der auf das Pixel abgebildeten Gefäße 403, 404 und/oder Gefäßabschnitte 403.1, 403.2.

Der Konfidenzwert eines ersten Pixels wird bestimmt, indem die eindimensionale Röntgenintensitätsverteilung entlang eines Strahls ausgehend von der Röntgenquelle 301 zum ersten Pixel aus dem ersten dreidimensionalen DSA-Datensatz ermittelt wird. Die Zahl der auf das erste Pixel projizierten Gefäße 403, 404 und/oder Gefäßabschnitte 403.1, 403.2 ist dann die Zahl der durch diesen Strahl durchquerten Gefäße 403, 404 und/oder Gefäßabschnitte 403.1, 403.2. Die Zahl der durchquerten Gefäße 403, 404 und/oder Gefäßabschnitte 403.1, 403.2 kann anhand des Gradienten der Röntgenintensitätsverteilung ermittelt werden, der Ränder von Gefäßen 403, 404 und/oder Gefäßabschnitten 403.1, 403.2 anzeigt. In die Ermittlung der Zahl der durchquerten Gefäße 403, 404 und/oder Gefäßabschnitte 403.1, 403.2 können weiterhin auch Erfahrungswerte zur durchschnittlichen Größe eines Gefäßes 403, 404 und/oder Gefäßabschnittes 403.1, 403.2 einfließen. Alternativ ist es auch möglich, den ersten dreidimensionalen DSA-Datensatz zu segmentieren, und die Zahl der durchquerten Gefäße 403, 404 und/oder Gefäßabschnitte 403.1, 403.2 basierend auf der Segmentierung zu bestimmen. Alternativ ist es ebenfalls möglich, einen Konfidenzwert basierend auf der Anzahl der vom Strahl durchquerten Voxel des ersten dreidimensionalen DSA-Datensatzes zu bestimmen, wobei die Voxel einen Wert über einem Schwellenwert aufweisen.

Ein weiterer optionaler Schritt des dargestellten Ausführungsbeispiels des Verfahrens ist ein Zuordnen ASG des Konfidenzwertes zu einem ersten Voxel wenigstens eines der dritten dreidimensionalen DSA-Datensätze, wobei der Wert des ersten Voxels auf dem Wert des ersten Pixels basiert, wobei der erste Voxel dem ersten Pixel über die Rückprojektion zugeordnet ist. Der Wert des ersten Voxels eines der dritten dreidimensionalen DSA-Datensätze basiert dabei auf dem Wert des ersten Pixel, wenn der Aufnahmezeitpunkt der den ersten Pixel umfassenden Röntgenprojektion und die Zeitinformation des zum ersten Voxel zugehörigen dreidimensionalen DSA-Datensatz der dritten dreidimensionalen DSA-Datensätze einander entsprechen, und wenn der Strahl von der Röntgenquelle 301 zum ersten Pixel zum Aufnahmezeitpunkt durch das erste Voxel verläuft. Im dargestellten Ausführungsbeispiel wird der Konfidenzwert jedes Pixels jeder der Röntgenprojektionen den entsprechenden Voxeln der dritten dreidimensionalen DSA-Datensätze zugeordnet. Es ist aber auch möglich, die Zuordnung nur für eine begrenzte Zahl von Pixeln von Röntgenprojektionen 501.1, ..., 501.4 oder von Voxeln der dritten dreidimensionalen DSA-Datensätze vorzunehmen.

Ein weiterer optionaler Schritt des dargestellten Ausführungsbeispiels des Verfahrens ist ein Interpolieren INTP des vierdimensionalen DSA-Datensatzes basierend auf dem Konfidenzwert. Dabei wird der vierdimensionale DSA-Datensatz in dem gezeigten Ausführungsbeispiel derart interpoliert, dass Intensitätswerte derjenigen Voxel von denjenigen dritten dreidimensionalen DSA-Datensätzen interpoliert werden, denen ein Konfidenzwert kleiner als ein Schwellenwert zugeordnet ist. In diesem Ausführungsbeispiel wird als Schwellenwert der maximale Konfidenzwert gewählt, so dass für diejenigen Voxel interpoliert wird, denen aufgrund von Überlagerung von Gefäßen 403, 404 und/oder Gefäßabschnitten 403.1, 403.2 kein exakter Intensitätswert zugewiesen werden kann. Im dargestellten Ausführungsbeispiel erfolgt die Interpolation für ein erstes Voxel in einem der dritten dreidimensionalen DSA-Datensätze nur anhand der Intensitätswerte der räumlich entsprechenden Voxel der anderen der dritten dreidimensionalen DSA-Datensätze, insbesondere also nur eine zeitliche Interpolation. Dabei entsprechen sich zwei Voxel insbesondere räumlich, falls ihre Position durch die gleichen räumlichen Koordinaten beschrieben wird. Es ist aber auch möglich, die Intensitätswerte von anderen Voxel in die Interpolation alleine oder zusätzlich zu verwenden, und dadurch alleine oder zusätzlich eine räumliche Interpolation durchzuführen. Weiterhin erfolgt im dargestellten Ausführungsbeispiel eine zeitlich lineare Interpolation zwischen dem Intensitätswert eines räumlich entsprechenden zweiten Voxels und eines räumlich entsprechenden dritten Voxels als Stützstellen, wobei dem zweiten Voxel und dem dritten Voxel der maximale Konfidenzwert zugeordnet. Hierbei ist die dem zweiten Voxel zugeordnete zweite Zeitinformation kleiner als die dem ersten Voxel zugeordnete erste Zeitinformation. Weiterhin gibt es keinen anderen räumlich entsprechenden Voxel mit maximalem Konfidenzwert, dem eine Zeitinformation zwischen der zweiten Zeitinformation und der ersten Zeitinformation zugeordnet ist. Weiterhin ist hierbei die dem dritten Voxel zugeordnete dritte Zeitinformation größer als die dem ersten Voxel zugeordnete erste Zeitinformation. Weiterhin gibt es keinen anderen räumlich entsprechenden Voxel mit maximalem Konfidenzwert, dem eine Zeitinformation zwischen der ersten Zeitinformation und der dritten Zeitinformation zugeordnet ist. Die Interpolation kann aber auch auf anderen oder weiteren Voxeln als Stützstellen basieren, weiterhin ist auch eine nichtlineare Interpolation, beispielsweise mittels Polynomen oder Polynomzügen, möglich.

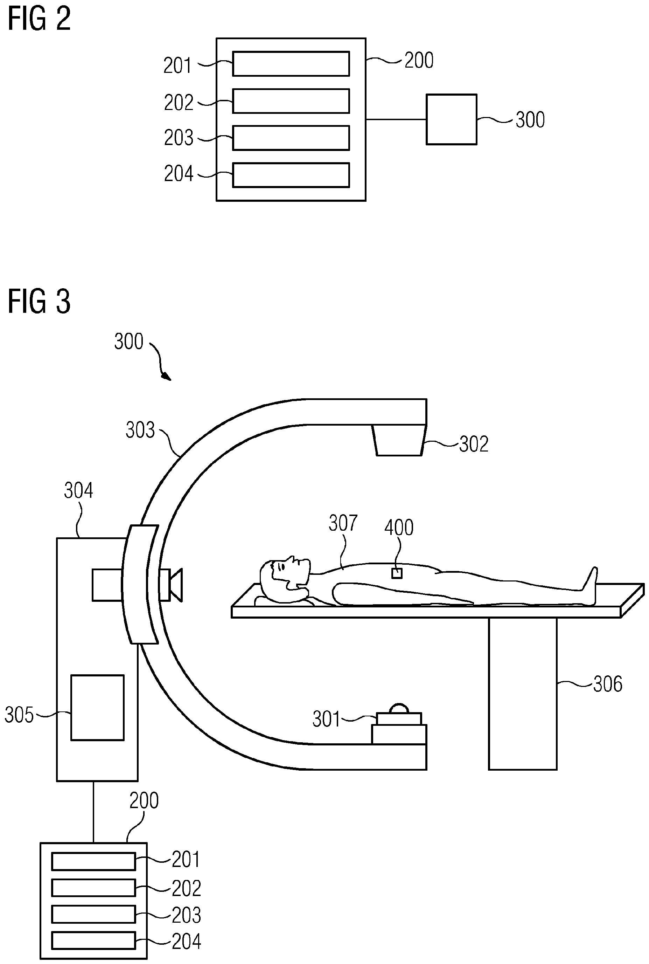

Fig. 2 zeigt eine DSA-Berechnungseinheit 200 zur Berechnung eines vierdimensionalen DSA-Datensatzes.

Die hier gezeigte DSA-Berechnungseinheit 200 ist ausgelegt, ein erfindungsgemäßes Verfahren auszuführen. Diese DSA-Berechnungseinheit 200 umfasst eine Schnittstelle 201, eine Recheneinheit 202, eine Speichereinheit 203 sowie eine Ein- und Ausgabeeinheit 204.

Bei der DSA-Berechnungseinheit 200 kann es sich insbesondere um einen Computer, einen Mikrocontroller oder um einen integrierten Schaltkreis handeln. Alternativ kann es sich bei der DSA-Berechnungseinheit 200 um einen realen oder virtuellen Verbund von Computern handeln (ein englischer Fachbegriff für einen realen Verbund ist "Cluster", ein englischer Fachbegriff für einen virtuellen Verbund ist "Cloud"). Bei einer Schnittstelle 201 kann es sich um eine Hardware- oder Softwareschnittstelle handeln (beispielsweise PCI-Bus, USB oder Firewire). Eine Recheneinheit 202 kann Hardware-Element oder Software-Elemente aufweisen, beispielsweise einen Mikroprozessor oder ein sogenanntes FPGA (englisches Akronym für "Field Programmable Gate Array"). Eine Speichereinheit 203 kann als nicht dauerhafter Arbeitsspeicher (Random Access Memory, kurz RAM) oder als dauerhafter Massenspeicher (Festplatte, USB-Stick, SD-Karte, Solid State Disk) realisiert sein. Eine Ein- und Ausgabeeinheit 204 umfasst wenigstens eine Eingabeeinheit und/oder wenigstens eine Ausgabeeinheit.

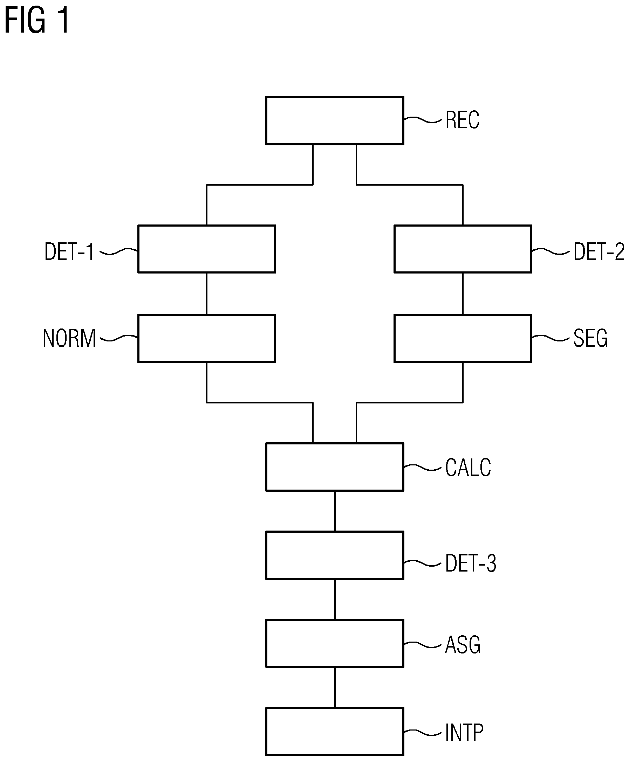

Im gezeigten Ausführungsbeispiel ist die DSA-Berechnungseinheit 200 mit einer Röntgeneinheit 300 verbunden. Die Verbindung zur Röntgeneinheit 300 kann aber auch mittels eines Netzwerks hergestellt werden, beispielsweise ein Intranet oder das Internet. Die DSA-Berechnungseinheit 200 kann aber auch ein Teil Röntgeneinheit 300 sein. Die hier gezeigte DSA-Berechnungseinheit 200 ist dazu ausgebildet, das in Fig. 1 dargestellte Verfahren auszuführen, indem die Schnittstelle 201 und die Recheneinheit 202 dazu ausgebildet sind, die jeweiligen Schritte des Verfahrens auszuführen.

Fig. 3 zeigt Röntgeneinheit 300 verbunden mit einer DSA-Berechnungseinheit 200. Im gezeigten Ausführungsbeispiel handelt es sich bei der Röntgeneinheit 300 um ein C-Bogen-Röntgengerät 300. Das C-Bogen-Röntgengerät 300 umfasst eine Röntgenquelle 301 zum Aussenden von Röntgenstrahlen. Weiterhin umfasst das C-Bogen-Röntgengerät 300 einen Röntgendetektor 302 zum Empfangen von Röntgenstrahlen. Die Röntgenquelle 301 sowie der Röntgendetektor 302 sind an den zwei unterschiedlichen Enden des C-Bogens 303 befestigt. Der C-Bogen 303 des C-Bogen-Röntgengeräts 300 ist an einem Stativ 304 befestigt. Das Stativ 304 umfasst Antriebselemente, die dazu ausgelegt sind, die Position des C-Bogens 303 zu verändern. Insbesondere kann der C-Bogen 303 um zwei verschiedene Achsen gedreht werden. Das C-Bogen-Röntgengerät umfasst weiterhin eine Steuer- und Auswerteeinheit 305 sowie eine Patientenlagerungsvorrichtung 306, auf der ein Patient 307 gelagert werden kann. Mittels der Steuer- und Auswerteeinheit 305 kann die Position des C-Bogens 303 eingestellt werden und der C-Bogen 303 um das Untersuchungsvolumen 400 rotiert werden. Weiterhin können mittels der Steuer- und Auswerteeinheit 305 zweidimensionale Röntgenprojektionen des ersten Untersuchungsvolumens 400 aufgenommen und ausgewertet werden. Es ist alternativ zum dargestellten Ausführungsbeispiel auch möglich, dass die DSA-Berechnungseinheit 200 als Teil der Steuer- und Auswerteeinheit 305 ausgeführt ist.

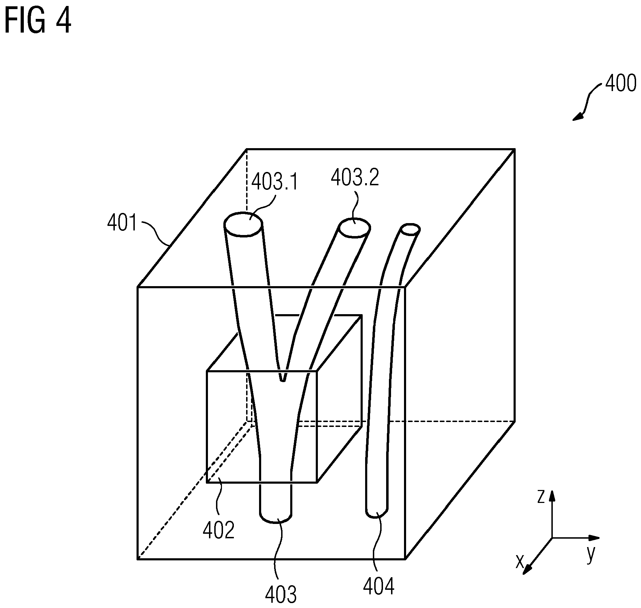

Fig. 4 zeigt ein Untersuchungsvolumen 400 mit einem ersten Gefäß 403 und einem zweiten Gefäß 404, wobei das Untersuchungsvolumen weiterhin ein erstes Rekonstruktionsvolumen 401 und ein zweites Rekonstruktionsvolumen 402 umfasst. Im dargestellten Ausführungsbeispiel ist das erste Rekonstruktionsvolumen 401 mit dem Untersuchungsvolumen 400 identisch, es ist aber auch vorstellbar, dass das erste Rekonstruktionsvolumen 401 kleiner als das Untersuchungsvolumen 400 ist und im Untersuchungsvolumen 400 enthalten ist. Weiterhin ist das zweite Rekonstruktionsvolumen 402 kleiner als das erste Rekonstruktionsvolumen 401, und das erste Gefäß 403 spaltet sich in einen ersten Gefäßabschnitt 403.1 und einen zweiten Gefäßabschnitt 403.2. Das zweite Gefäß 404 verläuft in dem gezeigten Beispiel außerhalb des zweiten Rekonstruktionsvolumens 402, aber innerhalb des ersten Rekonstruktionsvolumens 401.

Das erste Rekonstruktionsvolumen 401 und das zweite Rekonstruktionsvolumen 402 sind hier würfelförmig ausgebildet, es sind aber auch andere geometrische Formen des ersten Rekonstruktionsvolumens 401 und des zweiten Rekonstruktionsvolumens 402 vorstellbar. Die Kanten des würfelförmigen ersten Rekonstruktionsvolumens 401 und des würfelförmigen zweiten Rekonstruktionsvolumens 402 sind hier parallel zu einer ersten Koordinatenachse X, einer zweiten Koordinatenachse Y oder einer dritten Koordinatenachse Z. Die erste Koordinatenachse X, die zweite Koordinatenachse Y und die dritte Koordinatenachse Z bilden im gezeigten Ausführungsbeispiel ein rechtshändiges, karthesisches Koordinatensystem.

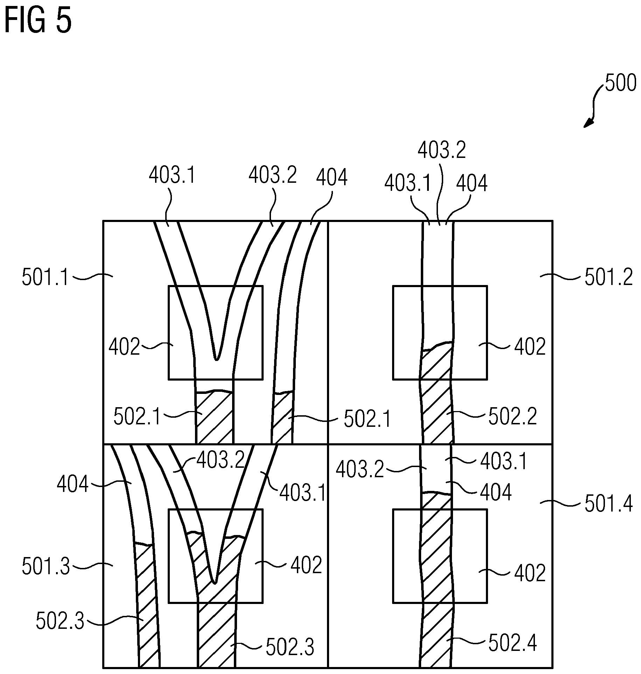

Fig. 5 zeigt Röntgendatensätze 500 jeweils umfassend eine der vier Röntgenprojektionen 501.1, 501.2, 501.3 und 501.4, die zu unterschiedlichen Aufnahmezeitpunkten aufgenommen wurden. Die Röntgenprojektionen 501.1, ..., 501.4 sind jeweils Projektionen des Untersuchungsvolumens 400 und damit des ersten Rekonstruktionsvolumens 401 aus unterschiedlichen Richtungen. In den Röntgenprojektionen 501.1, ..., 501.4 ist jeweils auch die Projektion des zweiten Rekonstruktionsvolumens 402 dargestellt.

Die erste Röntgenprojektion 501.1 ist eine Projektion des Untersuchungsvolumens 400 entgegen der Richtung der ersten Koordinatenachse X. Die zweite Röntgenprojektion 501.2 ist eine Projektion des Untersuchungsvolumens 400 in Richtung der zweiten Koordinatenachse Y und wurde zeitlich nach der ersten Röntgenprojektion 501.1 aufgenommen. Die dritte Röntgenprojektion 501.3 ist eine Projektion des Untersuchungsvolumens 400 in Richtung der ersten Koordinatenachse X und wurde zeitlich nach der zweiten Röntgenprojektion 501.2 aufgenommen. Die vierte Röntgenprojektion 501.4 ist eine Projektion des Untersuchungsvolumens 400 entgegen der Richtung der zweiten Koordinatenachse Y und wurde zeitlich nach der dritten Röntgenprojektion 501.3 aufgenommen.

Die Röntgenprojektionen 501.1, ..., 501.4 enthalten jeweils Projektionen des ersten Gefäßes 403 und des zweiten Gefäßes 404. In der zweiten Röntgenprojektion 501.2 und in der vierten Röntgenprojektion 501.4 überlappen aufgrund der jeweiligen Projektionsrichtung das erste Gefäß 403 und das zweite Gefäß 404 derart, dass sie nicht einzeln in der Röntgenprojektion 501.2, 501.4 dargestellt werden können.

Weiterhin sind in den Röntgenprojektionen 501.1, ..., 501.4 Bereiche 502.1, ..., 502.4 mit hoher Intensität dargestellt. In diesem Ausführungsbeispiel entsprechen die Bereiche 502.1, ..., 502.4 Teilen des ersten Gefäßes 403 und des zweiten Gefäßes 404, die mit einem Kontrastmittel gefüllt sind. Da die Röntgenprojektionen 501.1, ..., 501.4 zu unterschiedlichen Aufnahmezeitpunkten aufgenommen sind, unterscheidet sich die Ausdehnung der Bereiche 502.1, ..., 502.4 mit hoher Intensität in den einzelnen Röntgenprojektionen 501.1, ..., 501.4.

Verwendet man wie aus dem Stand der Technik bekannt zur Bestimmung des vierdimensionalen DSA-Datensatzes nur ein Rekonstruktionsvolumen, so ergeben sich in der dargestellten Gefäßstruktur zwei Optionen. Einerseits kann man als Rekonstruktionsvolumen das erste Rekonstruktionsvolumen 401 verwenden, in dem neben dem ersten Gefäß 403 auch das zweite Gefäß 404 enthalten ist, und so den Beitrag des zweiten Gefäßes zu den Röntgenprojektionen 501.1, ..., 501.4 der Röntgendatensätze 500 zumindest mittels einer Interpolation bestimmen. Der vierdimensionale DSA-Datensatz hat in diesem Fall aber nur eine grobe räumliche Auflösung. Andererseits kann man als Rekonstruktionsvolumen das zweite Rekonstruktionsvolumen 402 verwenden, in dem nur das erste Gefäß 403 enthalten ist, und so eine feinere räumliche Auflösung des vierdimensionalen DSA-Datensatzes erhalten. In diesem Fall kann man aber den Beitrag des zweiten Gefäßes 404 zu den Röntgenprojektionen 501.1, ..., 501.4 des der Röntgendatensätze 500 nicht rechnerisch bestimmen. In einem erfindungsgemäßen Verfahren ist es aber möglich, beide Vorteile ohne die jeweiligen Nachteile zu erreichen.

## Patentansprüche

1. Verfahren zum Berechnen eines vierdimensionalen DSA-Datensatzes aus Röntgendatensätzen (500), umfassend folgende Verfahrensschritte:

    - Empfangen (REC) von Röntgendatensätzen (500) betreffend ein Untersuchungsvolumen (400) mittels einer Schnittstelle (201), wobei jeder der Röntgendatensätze (500) eine zweidimensionale Röntgenprojektion (501.1, 501.2, 501.3, 501.4) des Untersuchungsvolumens (400) bezüglich einer Projektionsrichtung und einen Aufnahmezeitpunkt der Röntgenprojektion (501.1, 501.2, 501.3, 501.4) umfasst,
    - Erstes Bestimmen (DET-1) eines ersten dreidimensionalen DSA-Datensatzes eines ersten Rekonstruktionsvolumens (401) basierend auf den Röntgendatensätzen (500) mittels einer Recheneinheit (202), wobei das erste Rekonstruktionsvolumen (401) ein Teil des Untersuchungsvolumens (400) oder mit diesem identisch ist,
    - Zweites Bestimmen (DET-2) eines zweiten dreidimensionalen DSA-Datensatzes eines zweiten Rekonstruktionsvolumens (402) basierend auf den Röntgendatensätzen (500) mittels der Recheneinheit (202), wobei das zweite Rekonstruktionsvolumen (402) ein Teil des ersten Rekonstruktionsvolumens (401) ist,
    - Segmentieren (SEG) des zweiten dreidimensionalen DSA-Datensatzes mittels der Recheneinheit (202),
    - Normieren (NORM) der Röntgendatensätze (500) basierend auf dem ersten dreidimensionalen DSA-Datensatz mittels der Recheneinheit (202),
    - Berechnen (CALC) eines vierdimensionalen DSA-Datensatzes durch Rückprojektion der normierten Röntgendatensätze (500) auf den segmentierten zweiten dreidimensionalen DSA-Datensatz mittels der Recheneinheit (202), wobei der vierdimensionale DSA-Datensatz mehrere dritte dreidimensionale DSA-Datensätze sowie eine zugehörige Zeitinformation umfasst.

2. Verfahren nach Anspruch 1, wobei jeder dritte dreidimensionale DSA-Datensatz des vierdimensionalen DSA-Datensatzes durch Rückprojektion von genau einem der zweidimensionalen Röntgendatensätze berechnet wird, und wobei die zugehörige Zeitinformation dem Aufnahmezeitpunkt des zweidimensionalen Röntgendatensatzes entspricht.

3. Verfahren nach einem der vorherigen Ansprüche, weiterhin umfassend folgende Verfahrensschritte, jeweils durchgeführt mittels der Recheneinheit (202):

    - Drittes Bestimmen (DET-3) eines Konfidenzwertes für wenigstens ein erstes Pixel wenigstens einer der Röntgenprojektionen (501.1, 501.2, 501.3, 501.4) basierend auf dem ersten dreidimensionalen DSA-Datensatz,
- Zuordnen (ASG) des Konfidenzwertes zu einem ersten Voxel wenigstens eines der dritten dreidimensionalen DSA-Datensätze,
wobei der Wert des ersten Voxels auf dem Wert des ersten Pixels basiert,
- Interpolieren (INTP) des vierdimensionalen DSA-Datensatzes basierend auf dem Konfidenzwert.

4. Verfahren nach Anspruch 3, wobei der Konfidenzwert des ersten Pixels einer Röntgenprojektion (501.1, 501.2, 501.3, 501.4) monoton mit einer Anzahl der auf den ersten Pixel projizierten Gefäßabschnitte (403.1, 403.2, 404) in dem ersten dreidimensionalen DSA-Datensatz sinkt, wobei die Gefäßabschnitte (403.1, 403.2, 404) in die Projektionsrichtung der Röntgenprojektion (501.1, 501.2, 501.3, 501.4) projiziert werden.

5. Verfahren nach Anspruch 4, wobei das Interpolieren Voxel betrifft, denen ein Konfidenzwert kleiner als ein Schwellenwert zugeordnet ist.

6. Verfahren nach einem der vorherigen Ansprüche, wobei der erste und der zweite dreidimensionale DSA-Datensatz jeweils homogene Voxel umfassen.

7. Verfahren nach Anspruch 6, wobei die Orientierung der Voxel im ersten dreidimensionalen DSA-Datensatz der Orientierung der Voxel im zweiten dreidimensionalen DSA-Datensatz entspricht.

8. Verfahren nach Anspruch 6 oder 7, wobei die Länge der bezüglich einer ersten Koordinatenachse (X) parallelen Kanten der Voxel des zweiten dreidimensionalen DSA-Datensatzes kleiner ist als die Länge der bezüglich der ersten Koordinatenachse (X) parallelen Kanten der Voxel des ersten dreidimensionalen DSA-Datensatzes.

9. Verfahren nach einem der Ansprüche 6 bis 8, wobei die Zahl der Voxel im ersten dreidimensionalen DSA-Datensatz gleich der Zahl der Voxel im zweiten dreidimensionalen DSA-Datensatz ist.

10. Verfahren nach einem der Ansprüche 6 bis 9, wobei die Kantenlängen der Voxel des zweiten dreidimensionalen DSA-Datensatzes größer sind als die Kantenlänge der Pixel der Röntgendatensätze (500).

11. DSA-Berechnungseinheit (200) zum Berechnen eines vierdimensionalen DSA-Datensatzes, umfassend folgende Einheiten:

- Schnittstelle (201), ausgebildet zum Empfangen (REC) von Röntgendatensätzen (500) betreffend ein Untersuchungsvolumen (400), wobei jeder der Röntgendatensätze (500) eine zweidimensionale Röntgenprojektion (501.1, 501.2, 501.3, 501.4) des Untersuchungsvolumens (400) bezüglich einer Projektionsrichtung und einen Aufnahmezeitpunkt der Röntgenprojektion (501.1, 501.2, 501.3, 501.4) umfasst,
- Recheneinheit (202), ausgebildet zum ersten Bestimmen (DET-1) eines ersten dreidimensionalen DSA-Datensatzes eines ersten Rekonstruktionsvolumens (401) basierend auf den Röntgendatensätzen (500), wobei das erste Rekonstruktionsvolumen (401) ein Teil des Untersuchungsvolumens (400) oder mit diesem identisch ist,

weiterhin ausgebildet zum zweiten Bestimmen (DET-2) eines zweiten dreidimensionalen DSA-Datensatzes eines zweiten Rekonstruktionsvolumens (402) basierend auf den Röntgendatensätzen (500), wobei das zweite Rekonstruktionsvolumen (402) ein Teil des ersten Rekonstruktionsvolumens (401) ist,
weiterhin ausgebildet zum Segmentieren (SEG) des zweiten dreidimensionalen DSA-Datensatzes,
weiterhin ausgebildet zum Normieren (NORM) der Röntgendatensätze (500) basierend auf dem ersten dreidimensionalen DSA-Datensatz,
weiterhin ausgebildet zum Berechnen (CALC) eines vierdimensionalen DSA-Datensatzes durch Rückprojektion der normierten Röntgendatensätze (500) auf den segmentierten zweiten dreidimensionalen DSA-Datensatz, wobei der vierdimensionale DSA-Datensatz mehrere dritte dreidimensionale DSA-Datensätze sowie eine zugehörige Zeitinformation umfasst.

12. DSA-Berechnungseinheit (200) nach Anspruch 11, weiterhin ausgebildet ein Verfahren nach den Ansprüchen 2 bis 10 auszuführen.

13. Röntgeneinheit (300), ausgebildet zum Aufnehmen von Röntgendatensätzen (500), umfassend eine DSA-Berechnungseinheit (200) nach Anspruch 10 oder 11.

14. Computerprogrammprodukt mit einem Computerprogramm, welches direkt in einen Speicher (203) einer DSA-Berechnungseinheit (200) ladbar ist, mit Programmabschnitten, um alle Schritte des Verfahrens nach einem der Ansprüche 1 bis 10 auszuführen, wenn die Programmabschnitte von der DSA-Berechnungseinheit (200) ausgeführt werden.

15. Computerlesbares Speichermedium, auf welchem von einer DSA-Berechnungseinheit (200) lesbare und ausführbare Programmabschnitte gespeichert sind, um alle Schritte des Verfahrens nach einem der Ansprüche 1 bis 10 auszuführen, wenn die Programmabschnitte von der DSA-Berechnungseinheit (200) ausgeführt werden.

**Claims**

1. Method for calculating a four-dimensional DSA dataset from x-ray datasets (500), comprising the following method steps:

   - Receiving (REC) x-ray datasets (500) relating to an examination volume (400) by means of an interface (201), wherein each of the x-ray datasets (500) comprises a two-dimensional x-ray projection (501.1, 501.2, 501.3, 501.4) of the examination volume (400) in relation to a direction of projection and a recording time of the x-ray projection (501.1, 501.2, 501.3, 501.4),
   - First determination (DET-1) of a first three-dimensional DSA dataset of a first reconstruction volume (401) on the basis of the x-ray datasets (500) by means of a calculation unit (202), wherein the first reconstruction volume (401) is a part of the examination volume (400) or is identical to said volume,
   - Second determination (DET-2) of a second three-dimensional DSA dataset of a second reconstruction volume (402) on the basis of the x-ray datasets (500) by means of the calculation unit (202),
   wherein the second reconstruction volume (402) is a part of the first reconstruction volume (401),
   - Segmentation (SEG) of the second three-dimensional DSA dataset by means of the calculation unit (202),
   - Normalization (NORM) of the x-ray datasets (500) on the basis of the first three-dimensional DSA dataset by means of the calculation unit (202),
   - Calculation (CALC) of a four-dimensional DSA dataset by back projection of the normalized x-ray datasets (500) onto the segmented second three-dimensional DSA dataset by means of the calculation unit (202),
   wherein the four-dimensional DSA dataset comprises a number of third three-dimensional DSA datasets as well as an associated item of time information.

2. Method according to claim 1, wherein each third three-dimensional DSA dataset of the four-dimensional DSA dataset is calculated by back projection from precisely one of the two-dimensional x-ray datasets, and wherein the associated time information corresponds to the recording time of the two-dimensional x-ray dataset.

3. Method according to one of the preceding claims, furthermore comprising the following method steps, each carried out by means of the calculation unit (202):

   - Third determination (DET-3) of a confidence value for at least one first pixel of at least one of the x-ray projections (501.1, 501.2, 501.3, 501.4) on the basis of the first three-dimensional DSA dataset,
   - Assignment (ASG) of the confidence value to a first voxel of at least one of the third three-dimensional DSA datasets, wherein the value of the first voxel is based on the value of the first pixel,
   - Interpolation (INTP) of the four-dimensional DSA dataset on the basis of the confidence value.

4. Method according to claim 3, wherein the confidence value of the first pixel of an x-ray projection (501.1, 501.2, 501.3, 501.4) falls monotonously with a number of the vessel sections (403.1, 403.2, 404) in the first three-dimensional DSA dataset projected onto the first pixel, wherein the vessel sections (403.1, 403.2, 404) are projected in the direction of projection of the x-ray projection (501.1, 501.2, 501.3, 501.4).

5. Method according to claim 4, wherein the interpolation relates to voxels to which a confidence value smaller than a threshold value is assigned.

6. Method according to one of the preceding claims, wherein the first and the second three-dimensional DSA dataset comprise homogeneous voxels in each case.

7. Method according to claim 6, wherein the orientation of the voxels in the first three-dimensional DSA dataset corresponds to the orientation of the voxels in the second three-dimensional DSA dataset.

8.  Method according to claim 6 or 7, wherein the length of the edges of the voxels of the second three-dimensional DSA dataset parallel in relation to a first coordinate axis (X) is smaller than the length of the edges of the voxels of the first three-dimensional DSA dataset parallel in relation to the first coordinate axis (X).

9.  Method according to one of claims 6 to 8, wherein the number of the voxels in the first three-dimensional DSA dataset is equal to the number of the voxels in the second three-dimensional DSA dataset.

10. Method according to one of claims 6 to 9, wherein the edge lengths of the voxels of the second three-dimensional DSA dataset are greater than the edge length of the pixels of the x-ray datasets (500).

11. DSA calculation unit (200) for calculating a four-dimensional DSA dataset, comprising the following units:

    - An interface (201), embodied for receiving (REC) x-ray datasets (500) relating to an examination volume (400), wherein each of the x-ray datasets (500) comprises a two-dimensional x-ray projection (501.1, 501.2, 501.3, 501.4) of the examination volume (400) in relation to a direction of projection and a recording time of the x-ray projection (501.1, 501.2, 501.3, 501.4),
    - A calculation unit (202), embodied for first determination (DET-1) of a first three-dimensional DSA dataset of a first reconstruction volume (401) on the basis of the x-ray datasets (500), wherein the first reconstruction volume (401) is a part of the examination volume (400) or is identical with said volume,

    furthermore embodied for second determination (DET-2) of a second three-dimensional DSA dataset of a second reconstruction volume (402) on the basis of the x-ray datasets (500), wherein the second reconstruction volume (402) is a part of the first reconstruction volume (401),
    further embodied for segmentation (SEG) of the second three-dimensional DSA dataset,
    furthermore embodied for normalization (NORM) of the x-ray datasets (500) on the basis of the first three-dimensional DSA dataset,
    furthermore embodied for calculation (CALC) of a four-dimensional DSA dataset by back projection of the normalized x-ray datasets (500) onto the segmented second three-dimensional DSA dataset, wherein the four-dimensional DSA dataset comprises a number of third three-dimensional DSA datasets as well as an associated item of time information.

12. DSA calculation unit (200) according to claim 11, further embodied for carrying out a method according to claims 2 to 10.

13. X-ray unit (300), embodied for recording x-ray datasets (500), comprising a DSA calculation unit (200) according to claim 10 or 11.

14. Computer program product with a computer program that is able to be loaded directly into a memory (203) of a DSA calculation unit (200), with program sections for carrying out all steps of the method according to one of claims 1 to 10, when the program sections are executed by the DSA calculation unit (200).

15. Computer-readable storage medium, on which program sections able to be read and executed by a DSA calculation unit (200) are stored, in order to execute all steps of the method according to one of claims 1 to 10, when the program sections are executed by the DSA calculation unit (200).

**Revendications**

1.  Procédé de calcul d'un jeu de données DSA à quatre dimensions à partir de jeux (500) de données de rayons X, comprenant les stades de procédé suivants :

    - réception (REC) de jeux (500) de données de rayons X concernant un volume (400) à examiner au moyen d'une interface (201), chacun des jeux (500) de données de rayons X comprenant une projection (501.1, 501.2, 501.3, 501.4) de rayons X à deux dimensions du volume (400) à examiner suivant une direction de projection et un instant d'enregistrement de la projection (501.1, 501.2, 501.3, 501.4) de rayons X,
    - première détermination (DET-1) d'une premier jeu de données DSA à trois dimensions d'un premier volume (401) de reconstruction reposant sur les jeux (500) de données de rayons X au moyen d'une unité (202) de calcul, dans lequel le premier volume (401) de reconstruction est une partie du volume (400) à examiner ou y est identique,

- deuxième détermination (DET-2) d'un deuxième jeu de données DSA à trois dimensions d'un deuxième volume (402) de reconstruction reposant sur les jeux (500) de données de rayons X au moyen de l'unité (202) de calcul, le deuxième volume (402) de reconstruction étant une partie du premier volume (401) de reconstruction,
- segmentation (SEG) du deuxième jeu de données DSA à trois dimensions au moyen de l'unité (202) de calcul,
- normation (NORM) des jeux (500) de données de rayons X reposant sur le premier jeu de données DSA à trois dimensions au moyen de l'unité (202) de calcul,
- calcul (CALC) d'un jeu de données DSA à quatre dimensions par rétroprojection des jeux (500) de données de rayons X normés sur le deuxième jeu de données DSA à trois dimensions segmenté au moyen de l'unité (202) de calcul,

le jeu de données DSA à quatre dimensions comprenant plusieurs troisièmes jeux de données DSA à trois dimensions, ainsi qu'une information de temps associée.

2. Procédé suivant la revendication 1, dans lequel on calcule un jeu de données DSA à trois dimensions sur trois du jeu de données DSA à quatre dimensions par rétroprojection d'exactement l'un des jeux de données de rayons X à deux dimensions, l'information de temps associée correspondant à l'instant d'enregistrement du jeu de données de rayons X à deux dimensions.

3. Procédé suivant l'une des revendications précédentes, comprenant, en outre, les stades de procédé suivant, effectués chacun au moyen de l'unité (202) de calcul :

   - troisième détermination (DET-3) d'un niveau de confiance pour au moins un premier pixel d'au moins l'une des projections (501.1, 501.2, 501.3, 501.4) de rayons X reposant sur le premier jeu de données DSA à trois dimensions,
   - affectation (ASG) du niveau de confiance à un premier voxel d'au moins l'un des troisièmes jeux de données DSA à trois dimensions,
   la valeur du premier voxel reposant sur la valeur du premier pixel,
   - interpolation (INTP) du jeu de données DSA à quatre dimensions sur la base du niveau de confiance.

4. Procédé suivant la revendication 3, dans lequel le niveau de confiance du premier pixel d'une projection (501.1, 501.2, 501.3, 501.4) de rayons X s'abaisse de manière monotone avec un nombre des parties (403.1, 403.2, 404) de vaisseau, projetées sur le premier pixel, dans le premier jeu de données DSA à trois dimensions, les parties (403.1, 403.2, 404) de vaisseau étant projetées dans la direction de projection de la projection (501.1, 501.2, 501.3, 501.4) de rayons X.

5. Procédé suivant la revendication 4, dans lequel l'interpolation concerne des voxels auxquels est affecté un niveau de confiance plus petit qu'un niveau de seuil.

6. Procédé suivant l'une des revendications précédentes, dans lequel le premier et le deuxième jeu de données DSA à trois dimensions comprennent chacun des voxels homogènes.

7. Procédé suivant la revendication 6, dans lequel l'orientation des voxels du premier jeu de données DSA à trois dimensions correspond à l'orientation des voxels du deuxième jeu de données DSA à trois dimensions.

8. Procédé suivant la revendication 6 ou 7, dans lequel la longueur des bords, parallèles à un premier axe (X) de coordonnées, des voxels du deuxième jeu de données DSA à trois dimensions est plus petite que la longueur des bords, parallèles au premier axe (X) de coordonnées, des voxels des premiers jeux de données DSA à trois dimensions.

9. Procédé suivant l'une des revendications 6 à 8, dans lequel le nombre des voxels du premier jeu de données DSA à trois dimensions est égal au nombre des voxels du deuxième jeu de données DSA à trois dimensions.

10. Procédé suivant l'une des revendications 6 à 9, dans lequel les longueurs de bords des voxels du deuxième jeu de données DSA à trois dimensions sont plus grandes que les longueurs de bords des pixels des jeux (500) de données de rayons X.

**11.** Unité (200) de calcul DSA pour calculer un jeu de données DSA à quatre dimensions, comprenant des unités suivantes :

- une interface (201) constituée pour recevoir (REC) des jeux (500) de données de rayons X concernant un volume (400) à examiner, chacun des jeux (500) de données de rayons X comprenant une projection (501.1, 501.2, 501.3, 501.4) de rayons X à deux dimensions du volume (400) à examiner suivant une direction de projection et un instant d'enregistrement de la projection (501.1, 501.2, 501.3, 501.4) de rayons X,
- une unité (202) de calcul, constituée pour la première détermination (DET-1) d'un premier jeu de données DSA à trois dimensions d'un premier volume (401) de reconstruction, reposant sur les jeux (500) de données de rayons X, le premier volume (401) de reconstruction étant une partie du volume (400) à examiner ou y étant identique, constituée, en outre, pour la deuxième détermination (DET-2) d'un deuxième jeu de données DSA à trois dimensions d'un deuxième volume de reconstruction reposant sur les jeux (500) de données de rayons X, le deuxième volume (402) de reconstruction étant une partie du premier volume (401) de reconstruction,

constituée, en outre, pour la segmentation (SEG) du deuxième jeu de données DSA à trois dimensions,
constituée, en outre, pour la normation (NORM) des jeux (500) de données de rayons X sur la base du premier jeu de données DSA à trois dimensions,
constituée, en outre, pour le calcul (CALC) d'un jeu de données DSA à quatre dimensions par rétroprojection des jeux (500) de données de rayons X normés sur le deuxième jeu de données DSA à trois dimensions segmenté, le jeu de données DSA à quatre dimensions comprenant plusieurs troisièmes jeux de données DSA à trois dimensions, ainsi qu'une information de temps associée.

**12.** Unité (200) de calcul DSA suivant la revendication 11, constituée, en outre, pour exécuter un procédé suivant les revendications 2 à 10.

**13.** Unité (300) radiologique, constituée pour enregistrer des jeux (500) de données de rayons X, comprenant une unité (200) de calcul DSA suivant la revendication 10 ou 11.

**14.** Produit de programme d'ordinateur, comprenant un programme d'ordinateur, qui peut être chargé directement dans une mémoire (203) d'une unité (200) de calcul DSA, comprenant des parties de programme pour effectuer tous les stades du procédé suivant l'une des revendications 1 à 10, lorsque les parties de programme sont exécutées par l'unité (200) de calcul DSA.

**15.** Support de mémoire déchiffrable par ordinateur, sur lequel sont mises en mémoire des parties de programme déchiffrables et réalisables par une unité (200) de calcul DSA pour effectuer tous les stades du procédé suivant l'une des revendications 1 à 10, lorsque les parties de programme sont exécutées par l'unité (200) de calcul DSA.

## FIG 1

REC

DET-1

DET-2

NORM

SEG

CALC

DET-3

ASG

INTP

FIG 2

FIG 3

FIG 4

FIG 5

500

403.1    403.2    404      403.2
                          403.1 ) 404

501.1                                    501.2

402

502.1                  502.1        502.2

404    403.2           403.2    403.1
           403.1              404
501.3                  402                501.4

502.3              402

502.3                  502.4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **K. ROYALTY.** 4D DSA: New Methods and Applications for 3D Time-Resolved Angiography for C-arm CT Interventional Imaging. University of Wisconsin-Madison, 2014